# EUROPEAN PATENT SPECIFICATION

(11) **EP 4 301 217 B1**
(45) Date of publication and mention of the grant of the patent: **01.01.2025**
(21) Application number: 22715118.0
(22) Date of filing: 22.02.2022
(51) Int. Cl.: A61B 5/022, A61B 5/0225, A61B 5/00, A61B 8/08, A61B 8/00, G16H 50/30, A61B 5/024

(54) **AN APPARATUS AND A METHOD FOR MEASURING COMPLIANCE OF BLOOD VESSELS**
VORRICHTUNG UND VERFAHREN ZUR MESSUNG DER NACHGIEBIGKEIT VON BLUTGEFÄSSEN
APPAREIL ET PROCÉDÉ POUR MESURER LA SOUPLESSE DE VAISSEAUX SANGUINS

(30) Priority: 04.03.2021 FI 20215237
(43) Date of publication of application: 10.01.2024
(73) Proprietor: Turun yliopisto, 20014 Turun yliopisto (FI)
(72) Inventor: KAISTI, Matti, 20720 Turku (FI); PANULA, Tuukka, 20100 Turku (FI); SIRKIÄ, Jukka-Pekka, 20100 Turku (FI)
(74) Representative: Väänänen, Janne Kalervo
(86) International application number: PCT/FI2022/050116
(87) International publication number: WO 2022/184973

(56) References cited:
- WO-A1-2020/144397
- US-A1- 2016 367 154
- GOHICHI TANAKA ET AL: "A novel photoplethysmography technique to derive normalized arterial stiffness as a blood pressure independent measure in the finger vascular bed;Normalized arterial stiffness index in the finger vascular beds", PHYSIOLOGICAL MEASUREMENT, INSTITUTE OF PHYSICS PUBLISHING, BRISTOL, GB, vol. 32, no. 11, 26 October 2011 (2011-10-26), pages 1869 - 1883, XP020213466, ISSN: 0967-3334, DOI: 10.1088/0967-3334/32/11/003
- JING LIU ET AL: "Multi-Wavelength Photoplethysmography Enabling Continuous Blood Pressure Measurement With Compact Wearable Electronics", IEEE TRANSACTIONS ON BIOMEDICAL ENGINEERING, vol. 66, no. 6, 1 June 2019 (2019-06-01), USA, pages 1514 - 1525, XP055657857, ISSN: 0018-9294, DOI: 10.1109/TBME.2018.2874957

## Description

### Field of the disclosure

The disclosure relates to an apparatus and a method for measuring compliance of blood vessels, the measured compliance being indicative of stiffness of the blood vessels. Furthermore, the disclosure relates to a computer program for measuring compliance of blood vessels.

### Background

In many cases, increased blood vessel stiffness, especially arterial stiffness, occurs because of biological aging and arteriosclerosis. Increased arterial stiffness is associated with an increased risk of cardiovascular events such as myocardial infarction and stroke, which are two leading causes of death in the developed world. An increase in arterial stiffness also increases the load of the heart, since the heart needs to perform more work to maintain a required stroke volume. Over time, this increased workload causes left ventricular hypertrophy and left ventricular remodelling, which can lead to a heart failure. The increased workload may also be associated with a higher heart rate, a proportionately longer duration of systole, and a reduction of duration of diastole. This decreases the amount of time available for perfusion of cardiac tissue, which mainly occurs during diastole. Thus, a hypertrophic heart, which has a greater oxygen demand, may have a compromised supply of oxygen and nutrients. Increased arterial stiffness may also affect the time at which pulse wave reflections return to the heart. When a pulse wave travels through the circulation it undergoes reflection at sites where the transmission properties of the arterial tree change, i.e. at sites of flow impedance mismatch. These reflected waves propagate backwards towards the heart. The speed of propagation is increased in stiffer arteries and consequently reflected waves will arrive at the heart earlier in systole. This increases the load of the heart in systole.

Due to the reasons of the kind mentioned above, several techniques have been developed to estimate the stiffness of blood vessels. A commonly used method for measuring the arterial stiffness is based on pulse wave velocity "PWV", i.e. the speed at which an arterial pulse propagates along an artery. The PWV is indicative of the arterial stiffness because higher arterial stiffness corresponds to higher PWV in accordance with the Bramwell-Hill equation. The PWV can be calculated by measuring a pulse transit time and a distance travelled between two selected sites. A more detailed description of the method based on the PWV can be found e.g. in the publication Patrick Segers et al.: How to Measure Arterial Stiffness in Humans, Arteriosclerosis, Thrombosis, and Vascular Biology, Vol. 40, No. 5, pp. 1034-1043, 26 Dec 2019.

Publication Jukka-Pekka Sirkiä et al.: Multi-Wavelength Photoplethysmography Device for the Measurement of Pulse Transit Time in the Skin Microvasculature, Computing in Cardiology, 2020-09-16 describes a multi-wavelength photoplethysmography "MWPPG" device for studying the skin microvasculature. The device utilizes the fact that the penetration depth of light into the skin is depended on the light wavelength. Thus, the device allows to study blood vessels at different depths.

Publication US2017172430 describes a method for cuff-less blood pressure measurement. The method comprises recording a physiological signal and multi-wavelength photoplethysmography "PPG" signals from a predetermined body part, deriving the depth-specific PPG signal reflecting the arterial blood volume with the physiological signal as a reference, calculating the pulse transit time "PTT" from the physiological signal and the derived arterial blood PPG signal, and calculating the blood pressure from the calculated PTT and blood pressure relationship.

Publication US2019336016 describes a device for non-invasive capillary blood pressure measurement. The device comprises a front end in contact with a body to compress and decompress capillaries in tissue, a pressure control module for regulating contact pressure between the front end and the tissue, a pressure transducer coupled to the front end for measuring the contact pressure, a capillary sensing module for detecting capillary pulsations under the contact pressure modulation, and a computing system for running an algorithm to determine capillary pressure based on the capillary pulsations and the contact pressure modulation.

Publication Gohichi Tanaka et al: A novel photoplethysmography technique to derive normalized arterial stiffness as a blood pressure independent measure in the finger vascular bed; Normalized arterial stiffness index in the finger vascular beds, Physiological Measurement, Institute of Physics Publishing, Bristol, GB, vol. 32, no. 11, 26 October 2011, pages 1869-1883 describes a photoplethysmography technique to derive arterial stiffness so that the finger arterial elasticity index "FEI" is defined as a parameter n which denotes the curvilinearity of a descriptive exponential model of pressure P - volume Vₐ relationship: Vₐ = a - b e^{-nP}, where a and b are parameters of the descriptive exponential model.

Publication WO2020144397 describes an apparatus for measuring functionality of an arterial system of an individual. The apparatus comprises a photoplethysmography sensor for emitting, to the arterial system, electromagnetic radiation having a wavelength in the range from 475 nm to 600 nm and for receiving a part of the electromagnetic radiation reflected off the arterial system. The apparatus further comprises a pressure instrument for managing mechanical pressure applied on the arterial system when the photoplethysmography sensor emits and receives the electromagnetic radiation to and from the arterial system. The effect of the mechanical pressure on the envelope of the reflected electromagnetic radiation can be used for determining diastolic blood pressure of arteries or for determining whether there is normal endothelial function.

There is, however, still a need for techniques for measuring data indicative of the stiffness of blood vessels quickly and cost effectively.

### Summary

The following presents a simplified summary in order to provide a basic understanding of some aspects of various invention embodiments. The summary is not an extensive overview of the invention. It is neither intended to identify key or critical elements of the invention nor to delineate the scope of the invention. The following summary merely presents some concepts of the invention in a simplified form as a prelude to a more detailed description of exemplifying embodiments of the invention.

In accordance with the invention, there is provided a new apparatus for measuring compliance of blood vessels. The compliance is indicative of the stiffness of the blood vessels so that a lower compliance corresponds to a greater stiffness. An apparatus according to the invention comprises:
- a photoplethysmography "PPG" sensor configured to emit electromagnetic radiation to the blood vessels, to receive a part of the electromagnetic radiation reflected off the blood vessels, and to produce a measurement signal indicative of the received part of the electromagnetic radiation,
- a pressure instrument configured to produce mechanical pressure applied on the blood vessels, and
- a control system configured to control the pressure instrument to change, i.e. decrease or increase, the mechanical pressure linearly with respect to time t during emission of the electromagnetic radiation to the blood vessels and reception of the part of the electromagnetic radiation reflected off the blood vessels.

The control system is configured to find, from the measurement signal, a portion whose envelope, i.e. a curve outlining extremes of the measurement signal, has exponential change, i.e. exponential growth ~e^{αt} or exponential decrease ~e^{-αt}, with respect to time and to produce an estimate for a coefficient α of time related to the exponential change, the exponential change being exponential increase when the mechanical pressure is linearly decreased with respect to time, and the exponential change being exponential decrease when the mechanical pressure is linearly increased with respect to time. The coefficient of time is indicative of the compliance of the blood vessels and thereby indicative of the stiffness of the blood vessels, too.

The above-mentioned pressure instrument can be for example a device for directing mechanical pressure to a fingertip or a toe, or a device comprising a cuff and a pump system for controlling gas pressure inside the cuff to direct mechanical pressure to an arm. Thus, use of the apparatus according to the invention does not need e.g. measurements from different sites of a body unlike a method based on the pulse wave velocity "PWV".

The photoplethysmography "PPG" sensor can be configured to emit electromagnetic radiation having wavelength for example in the range from 625 nm to 1000 nm, i.e. red or infrared light, in order to measure compliance of arteries located in the hypodermis, and/or electromagnetic radiation having wavelength for example in the range from 565 nm to 590 nm, i.e. yellow light, in order to measure compliance of blood vessels located in an upper portion of the hypodermis, and/or electromagnetic radiation having wavelength for example in the range from 500 nm to 565 nm, i.e. green light, in order to measure compliance of arterioles located in the dermis, and/or electromagnetic radiation having wavelength for example in the range from 450 nm to 485 nm, i.e. blue light, in order to measure compliance of capillaries located in an upper portion of the dermis.

It is to be noted that the above-mentioned wavelengths are examples only and a measurement of compliance of blood vessels can be carried out with many different suitable wavelengths. A radiation emitter of a PPG sensor may comprise for example one of more light emitting diodes "LED" and/or laser sources. Furthermore, a continuum of compliance values can be measured using for example a radiation emitter having an adjustable wavelength.

In an exemplifying case where different waveforms are used, it is possible to measure the compliances of different blood vessels and thereafter compute a ratio of at least one pair of the measured compliances corresponding to different wavelengths, where each ratio expresses a stiffness mismatch between smaller and greater blood vessels wherein a shorter wavelength relates to the smaller blood vessels and a longer wavelength relates to the greater blood vessels.

In accordance with the invention, there is provided also a new method for measuring compliance of blood vessels. A method according to the invention comprises:
- emitting electromagnetic radiation to the blood vessels,
- receiving a part of the electromagnetic radiation reflected off the blood vessels,
- producing a measurement signal indicative of the received part of the electromagnetic radiation,
- producing mechanical pressure applied on the blood vessels and changing linearly with respect to time t during the emitting the electromagnetic radiation to the blood vessels and the receiving the part of the electromagnetic radiation reflected off the blood vessels,
- finding, from the measurement signal, a portion whose envelope has exponential change, ~e^{αt} or ~e^{-αt}, with respect to time, the exponential change being exponential increase when the mechanical pressure is linearly decreased with respect to time, and the exponential change being exponential decrease when the mechanical pressure is linearly increased with respect to time, and
- producing an estimate for a coefficient α of time related to the exponential change, the coefficient of time being indicative of the compliance of the blood vessels.

In accordance with the invention, there is provided also a new computer program for measuring compliance of blood vessels. A computer program according to the invention comprises computer executable instructions for controlling a programmable processing system to:
- control a photoplethysmography sensor to emit electromagnetic radiation to the blood vessels, to receive a part of the electromagnetic radiation reflected off the blood vessels, and to produce a measurement signal indicative of the received part of the electromagnetic radiation,
- control a pressure instrument to produce mechanical pressure applied on the blood vessels and to change the mechanical pressure linearly with respect to time t during emission of the electromagnetic radiation to the blood vessels and reception of the part of the electromagnetic radiation reflected off the blood vessels,
- find, from the measurement signal, a portion whose envelope has exponential change, ~e^{αt} or ~e^{-αt}, with respect to time, the exponential change being exponential increase when the mechanical pressure is linearly decreased with respect to time, and the exponential change being exponential decrease when the mechanical pressure is linearly increased with respect to time, and
- produce an estimate for a coefficient α of time related to the exponential change, the coefficient of time being indicative of the compliance of the blood vessels.

In accordance with the invention, there is provided also a new computer program product. A computer program product according to the invention comprises a nonvolatile computer readable medium, e.g. a compact disc "CD", encoded with a computer program according to the invention.

Exemplifying and non-limiting embodiments are described in accompanied dependent claims.

Various exemplifying and non-limiting embodiments both as to constructions and to methods of operation, together with additional objects and advantages thereof, will be best understood from the following description of specific exemplifying embodiments when read in conjunction with the accompanying drawings.

The verbs "to comprise" and "to include" are used in this document as open limitations that neither exclude nor require the existence of also un-recited features.

The features recited in the accompanied dependent claims are mutually freely combinable unless otherwise explicitly stated.

Furthermore, it is to be understood that the use of "a" or "an", i.e. a singular form, throughout this document does not exclude a plurality.

### Brief description of figures

Exemplifying and non-limiting embodiments and their advantages are explained in greater detail below with reference to the accompanying drawings, in which:
figure 1a illustrates an apparatus according to an exemplifying and non-limiting embodiment for measuring compliance of blood vessels,
figure 1b shows exemplifying graphs illustrating electromagnetic radiations having different wavelengths and reflected off blood vessels of a fingertip as a function of time,
figure 1c shows the exemplifying graphs of figure 1b converted to a logarithmic vertical scale,
figure 2 illustrates an apparatus according to an exemplifying and non-limiting embodiment for measuring compliance of blood vessels,
figure 3 shows a flowchart of a method according to an exemplifying and non-limiting embodiment for measuring compliance of blood vessels.

### Description of exemplifying and non-limiting embodiments

The specific examples provided in the description below should not be construed as limiting the scope and/or the applicability of the appended claims. Lists and groups of examples provided in the description are not exhaustive unless otherwise explicitly stated.

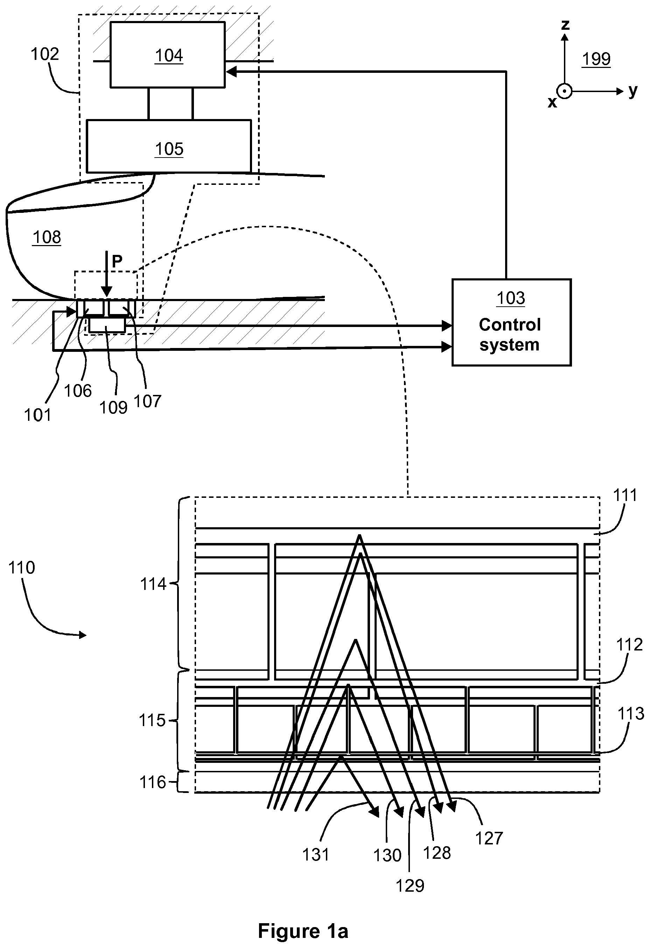

Figure 1a shows a schematic illustration of an apparatus according to an exemplifying and non-limiting embodiment for measuring compliance of blood vessels. The apparatus comprises a photoplethysmography "PPG" sensor 101 for emitting, to a fingertip 108 of an individual, electromagnetic radiation and for receiving a part of the electromagnetic radiation reflected off the blood vessels of the fingertip 108. The PPG sensor 101 comprises a radiation emitter 106 and a photodetector 107. The radiation emitter 106 may comprises e.g. one or more light emitting diodes "LED" and the photodetector 107 may comprise e.g. one or more photodiodes or phototransistors. Figure 1a shows also a magnified, schematic section view 110 of the fingertip. The section plane is parallel with the yz-plane of a coordinate system 199.

In the exemplifying apparatus illustrated in figure 1a, the PPG sensor 101 is configured to emit the electromagnetic radiation so that the electromagnetic radiation contains radiation components with five different wavelengths. In the section view 110, the radiation components are depicted with polyline arrows 127, 128, 129, 130, and 131. The first radiation component 127 can be for example infrared radiation having a wavelength on the range from 700 nm to 1000 nm, the second radiation component 128 can be for example red radiation having a wavelength on the range from 625 nm to 700 nm, the third radiation component 129 can be for example yellow radiation having a wavelength on the range from 565 nm to 590 nm, the fourth radiation component 130 can be for example green radiation having a wavelength on the range from 500 nm to 565 nm, and the fifth radiation component 131 can be for example blue radiation having a wavelength on the range from 450 nm to 485 nm. For another example, the first radiation component 127 can be infrared radiation having a wavelength on the range from 800 nm to 900 nm, the second radiation component 128 can be red radiation having a wavelength on the range from 650 nm to 675 nm, the third radiation component 129 can be yellow radiation having a wavelength on the range from 575 nm to 580 nm, the fourth radiation component 130 can be green radiation having a wavelength on the range from 530 nm to 545 nm, and the fifth radiation component 131 can be blue radiation having a wavelength on the range from 460 nm to 475 nm.

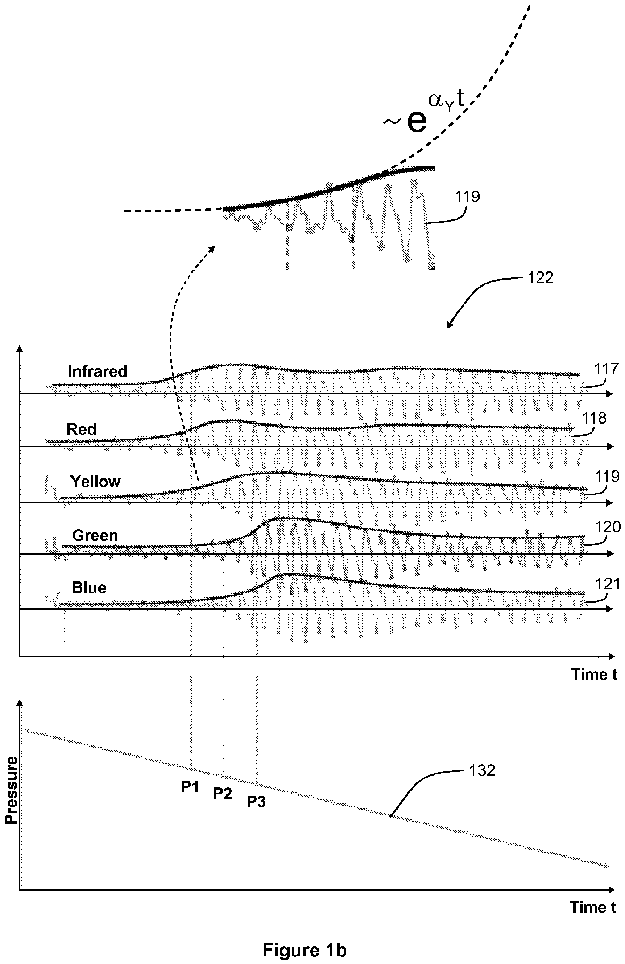

As illustrated in the section view 130, the red and infrared radiation components 127 and 128 reach arteries 111 located in the hypodermis 114, the yellow radiation component 129 reach blood vessels located in a portion of the hypodermis 114 adjacent to the dermis 115, the green radiation component 130 reach arterioles 112 located in the dermis 115, and the blue radiation component 131 reach capillaries 112 located in a portion of the dermis 115 adjacent to the epidermis 116. Therefore, shorter wavelengths relate to smaller blood vessels, i.e. blood vessels nearer to a skin surface, than longer wavelengths. In the exemplifying apparatus illustrated in figure 1a, the photodetector 107 of the PPG sensor 101 is configured produce a measurement signal that comprises wavelength-specific component signals indicative of received wavelengths reflected off the blood vessels. Figure 1b shows graphs illustrating exemplifying wavelength-specific component signals 117, 118, 119, 120, and 121 of an exemplifying measurement signal 122. The wavelength-specific component signal 117 corresponds to infrared radiation, the wavelength-specific component signal 118 corresponds to red radiation, the wavelength-specific component signal 119 corresponds to yellow radiation, the wavelength-specific component signal 120 corresponds to green radiation, and the wavelength-specific component signal 121 corresponds to blue radiation. The photodetector 107 may comprise for example many photodiodes or phototransistors that are sensitive to different wavelengths, or the photodetector 107 may comprise filters to implement wavelength separation.

The apparatus comprises a pressure instrument 102 configured to produce mechanical pressure P applied on the blood vessels. The apparatus comprises a control system 103 configured to control the pressure instrument 102 to change, i.e. to decrease or increase, the mechanical pressure linearly with respect to time t during emission of the electromagnetic radiation to the blood vessels and reception of the reflected electromagnetic radiation from the blood vessels. Figure 1b shows a line 132 illustrating the time dependence of the mechanical pressure in the exemplifying case in which the mechanical pressure has been linearly decreased and the above-mentioned wavelength-specific component signals 117-121 have been measured. The pressure values P1, P2, and P3 can be e.g. 127 mmHg, 100 mmHg, and 81 mmHg, respectively.

In the exemplifying apparatus illustrated in figure 1a, the pressure instrument 102 comprises a pressure sensor 109 for measuring the mechanical pressure P directed by the fingertip 108 to the pressure sensor 108 and pressing means for controllably pressing the fingertip 108 against the PPG sensor 101 and the pressure sensor 109. In this exemplifying apparatus, the pressing means comprise a pressing element 105 and a force generator 104 for directing force to the pressing element 105. The force generator 104 may comprise for example an electric stepper motor and a threaded rod or some other suitable elements for generating force.

The control system 103 is configured to find, from each wavelength-specific component of the measurement signal, a portion whose envelope has exponential change, i.e. exponential growth or exponential decrease, with respect to time and to produce an estimate for a coefficient of time related to the exponential change. The coefficient of time is indicative of the compliance of the blood vessels and thereby also the stiffness of the blood vessels.

As mentioned above, in the exemplifying case illustrated in figure 1b, the mechanical pressure is linearly decreased and thus each of the wavelength-specific component signals 117-121 has a portion whose envelope has exponential growth. Figure 1b shows a magnification of a part of the wavelength-specific component signal 119. In figure 1b, the envelopes of the wavelength-specific component signals 117-121 are depicted with thick lines. The exponential growth ~e^{αYt} on a part of the envelope of the wavelength-specific component signal 119 is depicted with a dashed line. In this case, the coefficient of time related to the exponential growth is α_{Y}. Thus, the coefficient of time α_{Y} is indicative of the compliance of the blood vessels and thereby also the stiffness of the blood vessels from which yellow light is reflected off.

There are many ways to find the portion whose envelope has the exponential change and to produce the estimate for the coefficient of time related to the exponential change. For example, curve fitting based on e.g. the least-mean-square "LMS" method can be used. Thus, apparatuses according to embodiments of the invention are not limited to any specific ways to find the portion whose envelope has the exponential change and to produce the estimate for the coefficient of time related to the exponential change.

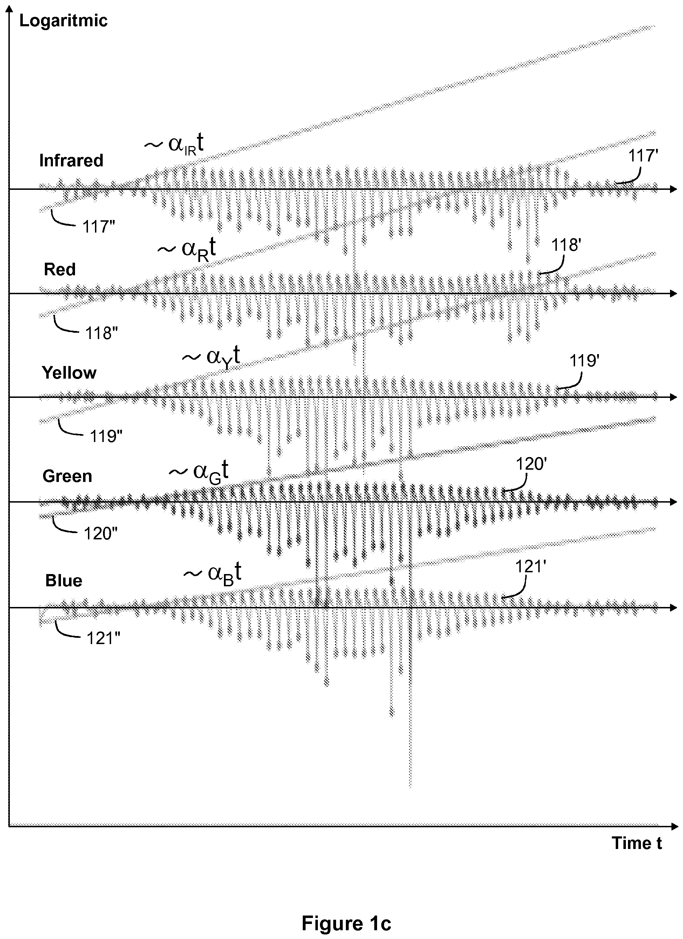

In an apparatus according to an exemplifying and non-limiting embodiment, the control system 103 is configured to convert the wavelength-specific components 117-121 of the measurement signal to a logarithmic scale. Figure 1c shows graphs illustrating the converted wavelength-specific component signals 117', 118', 119', 120', and 121' which corresponds to the wavelength-specific component signals 117, 118, 119, 120, and 121 shown in figure 1b, respectively. Due to the logarithmic conversion, the exponential growth is converted into linear growth that is depicted with lines 117", 118", 119", 120", and 121" shown in figure 1c. Concerning the linear growth, the control system 103 is configured to produce an estimate for a slope of the envelope of each converted wavelength-specific component signal. The slope is the coefficient of time related to the exponential growth. In the exemplifying case shown in figures 1b and 1c, the slope i.e. the coefficient of time related to the infrared light is α_{IR}, the coefficient of time related to the red light is α_{R}, the coefficient of time related to the yellow light is α_{Y}, the coefficient of time related to the green light is α_{G}, and the coefficient of time related to the blue light is α_{B}. Thus, for example, α_{R} is the indicative of compliance of arteries and thereby also the stiffness of the arteries, α_{G} is indicative of compliance of arterioles and thereby also the stiffness of the arterioles, and as is indicative of compliance of capillaries and thereby also the stiffness of the capillaries.

In an apparatus according to an exemplifying and non-limiting embodiment, the control system 103 is configured to compute a ratio of at least one pair of the coefficients of time corresponding to different wavelengths. Each ratio expresses a stiffness mismatch between blood vessels having different sizes where a shorter wavelength relates to smaller blood vessels and a longer wavelength relates to larger blood vessels. For example, the ratio α_{R}/α_{G} is indicative of the stiffness mismatch between arteries and arterioles.

In the exemplifying apparatus illustrated in figure 1a, the PPG sensor 101 is configured to emit and receive different wavelengths simultaneously. It is however also possible that the control system of an apparatus according to an exemplifying and non-limiting embodiment is configured to control the PPG sensor to variate the wavelength of the electromagnetic radiation, and to produce the coefficients of time for different wavelengths successively.

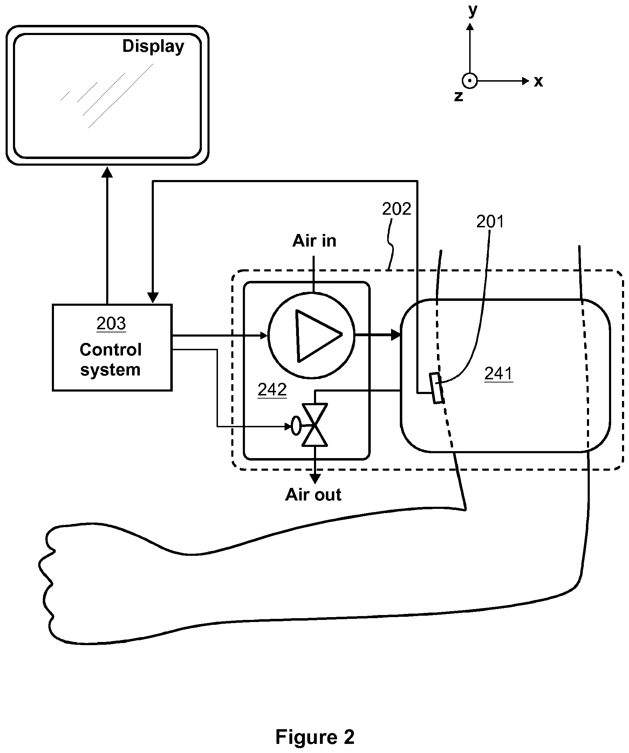

Figure 2 shows a schematic illustration of an apparatus according to an exemplifying and non-limiting embodiment for measuring compliance of blood vessels. The apparatus comprises a photoplethysmography "PPG" sensor 201 for emitting electromagnetic radiation and for receiving a part of the electromagnetic radiation reflected off blood vessels of an arm. The PPG sensor 201 is configured to produce a measurement signal indicative of the received part of the electromagnetic radiation. The apparatus comprises a pressure instrument 202 configured to produce mechanical pressure applied on the blood vessels. The apparatus comprises a control system 203 configured to control the pressure instrument 202 to change the mechanical pressure linearly with respect to time during emission of the electromagnetic radiation to the blood vessels and reception of the reflected electromagnetic radiation from the blood vessels. The control system 203 is configured to find, from the above-mentioned measurement signal, a portion whose envelope has exponential change, ~e^{αt} or ~e^{-αt}, with respect to time and to produce an estimate for a coefficient α of time related to the exponential change. The coefficient α of time is indicative of the compliance of the blood vessels and thereby indicative of the stiffness of the blood vessels.

In the exemplifying apparatus illustrated in figure 2, the pressure instrument 202 comprises a cuff and a pump system 242 configured to control gas pressure inside the cuff to direct the mechanical pressure to the arm and to change the mechanical pressure when the PPG sensor 201 emits and receives the electromagnetic radiation to and from the arm. The PPG sensor is located on an inner surface of the cuff.

Each of the control systems 103 and 203 shown in figures 1a and 2 can be implemented for example with one or more processor circuits, each of which can be a programmable processor circuit provided with appropriate software, a dedicated hardware processor such as for example an application specific integrated circuit "ASIC", or a configurable hardware processor such as for example a field programmable gate array "FPGA". Each of the control systems 103 and 203 may further comprise memory implemented for example with one or more memory circuits each of which can be e.g. a random-access memory "RAM" device.

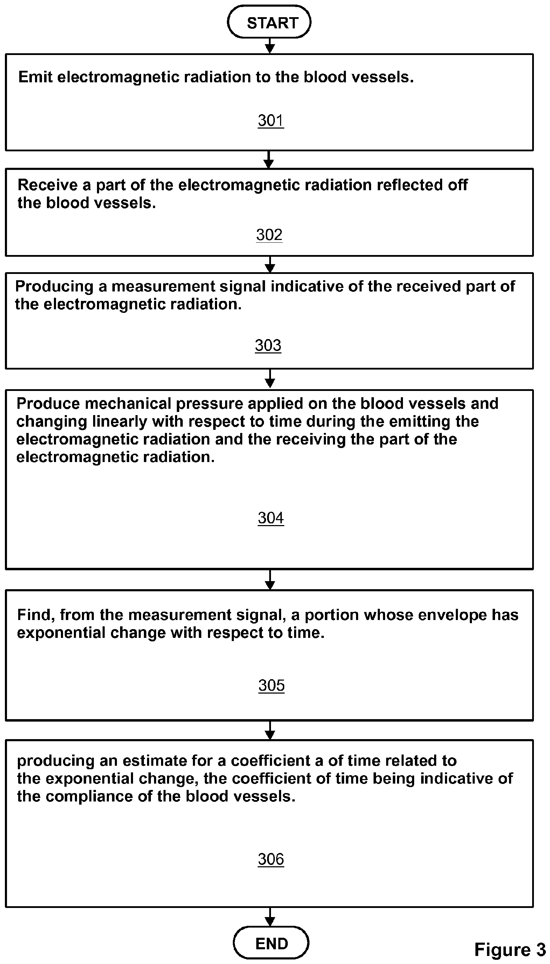

Figure 3 shows a flowchart of a method according to an exemplifying and non-limiting embodiment for measuring compliance of blood vessels. The method comprises the following actions:
- action 301: emitting electromagnetic radiation to the blood vessels,
- action 302: receiving a part of the electromagnetic radiation reflected off the blood vessels,
- action 303: producing a measurement signal indicative of the received part of the electromagnetic radiation,
- action 304: producing mechanical pressure applied on the blood vessels and changing linearly with respect to time t during the emitting the electromagnetic radiation to the blood vessels and the receiving the part of the electromagnetic radiation reflected off the blood vessels,
- action 305: finding, from the measurement signal, a portion whose envelope has exponential change, ~e^{αt} or ~e^{-αt}, with respect to time, and
- action 306: producing an estimate for a coefficient α of time related to the exponential change, the coefficient of time being indicative of the compliance of the blood vessels.

In a method according to an exemplifying and non-limiting embodiment, the electromagnetic radiation has wavelengths selected from at least two of the following ranges: from 625 nm to 1000 nm, from 565 nm to 590 nm, from 500 nm to 565 nm, and from 450 nm to 485 nm.

In a method according to an exemplifying and non-limiting embodiment, the electromagnetic radiation has different wavelengths and the measurement signal comprises wavelength-specific component signals being indicative of received wavelengths reflected off the blood vessels, and the coefficient of time is produced for each of the wavelength-specific component signals corresponding to the different wavelengths where a shorter one of the wavelengths relates to smaller ones of the blood vessels than a longer one of the wavelengths.

In a method according to an exemplifying and non-limiting embodiment, the wavelength of the electromagnetic radiation is varied, and the coefficient of time is produced for each value of the wavelength where a shorter wavelength relates to smaller ones of the blood vessels than a longer wavelength.

A method according to an exemplifying and non-limiting embodiment comprises computing a ratio of at least one pair of the coefficients of time corresponding to different wavelengths, each ratio expressing a stiffness mismatch between ones of the blood vessels having different sizes where a shorter wavelength relates to smaller ones of the blood vessels than a longer wavelength.

A method according to an exemplifying and non-limiting embodiment comprises converting the measurement signal to a logarithmic scale, finding from the converted measurement signal a portion whose envelope has linear change with respect to time, and producing an estimate for a slope of the envelope of the converted measurement signal related to the linear change. The slope of the linear change is the coefficient of time related to the exponential change.

In a method according to an exemplifying and non-limiting embodiment, the mechanical pressure is directed to a fingertip or a toe of an individual.

In a method according to an exemplifying and non-limiting embodiment, the mechanical pressure is directed to an arm of an individual with a cuff and a pump system configured to control gas pressure inside the cuff. In this exemplifying case, the photoplethysmography sensor is located on an inner surface of the cuff.

A computer program according to an exemplifying and non-limiting embodiment comprises computer executable instructions for controlling a programmable processing system to carry out actions related to a method according to any of the above-described exemplifying and non-limiting embodiments.

A computer program according to an exemplifying and non-limiting embodiment comprises software modules for measuring compliance of blood vessels. The software modules comprise computer executable instructions for controlling a programmable processing system to:
- control a photoplethysmography "PPG" sensor to emit electromagnetic radiation to the blood vessels, to receive a part of the electromagnetic radiation reflected off the blood vessels, and to produce a measurement signal indicative of the received part of the electromagnetic radiation,
- control a pressure instrument to produce mechanical pressure applied on the blood vessels and to change the mechanical pressure linearly with respect to time t during emission of the electromagnetic radiation to the blood vessels and reception of the part of the electromagnetic radiation reflected off the blood vessels,
- find, from the measurement signal, a portion whose envelope has exponential change, ~e^{αt} or ~e^{-αt}, with respect to time, and
- produce an estimate for a coefficient α of time related to the exponential change, the coefficient of time being indicative of the compliance of the blood vessels.

The software modules can be for example subroutines or functions implemented with programming tools suitable for the programmable processing equipment.

A computer program product according to an exemplifying and non-limiting embodiment comprises a computer readable medium, e.g. a compact disc "CD", encoded with a computer program according to an exemplifying embodiment.

A signal according to an exemplifying and non-limiting embodiment is encoded to carry information defining a computer program according to an exemplifying embodiment.

A computer program according to an exemplifying and non-limiting embodiment may constitute e.g. a part of a software of a mobile device, e.g. a smart phone or a wearable device.

The specific examples provided in the description given above should not be construed as limiting the scope and/or the applicability of the appended claims. Lists and groups of examples provided in the description given above are not exhaustive unless otherwise explicitly stated.

## Claims

1. apparatus for measuring compliance of blood vessels, the apparatus comprising:
- a photoplethysmography sensor (101, 201) configured to emit electromagnetic radiation to the blood vessels, to receive a part of the electromagnetic radiation reflected off the blood vessels, and to produce a measurement signal (122) indicative of the received part of the electromagnetic radiation,
- a pressure instrument (102, 202) configured to produce mechanical pressure applied on the blood vessels, and
- a control system (103, 203) configured to control the pressure instrument to change the mechanical pressure linearly with respect to time (t) during emission of the electromagnetic radiation to the blood vessels and reception of the part of the electromagnetic radiation reflected off the blood vessels,
**characterized in that** the control system is configured to find, from the measurement signal, a portion whose envelope has exponential change (~e^{αt} or ~e^{-αt}) with respect to time and to produce an estimate for a coefficient (α) of time related to the exponential change, the coefficient of time being indicative of the compliance of the blood vessels, the exponential change being exponential increase when the mechanical pressure is linearly decreased with respect to time, and the exponential change being exponential decrease when the mechanical pressure is linearly increased with respect to time.

2. An apparatus according to claim 1, wherein the photoplethysmography sensor (101) is configured to emit, to the blood vessels, the electromagnetic radiation so that the electromagnetic radiation has wavelengths selected from at least one of the following ranges: from 625 nm to 1000 nm, from 565 nm to 590 nm, from 500 nm to 565 nm, and from 450 nm to 485 nm.

3. An apparatus according to claim 1 or 2, wherein the photoplethysmography sensor (101) is configured to emit, to the blood vessels, the electromagnetic radiation so that the electromagnetic radiation has different wavelengths and to produce the measurement signal (122) to comprise wavelength-specific component signals (117-121) being indicative of received wavelengths reflected off the blood vessels, and the control system is configured to produce the coefficient of time for each of the wavelength-specific component signals corresponding to the different wavelengths where a shorter one of the wavelengths relates to smaller ones of the blood vessels than a longer one of the wavelengths.

4. An apparatus according to claim 1 or 2, wherein the control system (103) is configured to control the photoplethysmography sensor (101) to variate wavelength of the electromagnetic radiation, and to produce the coefficient of time for each value of the wavelength where a shorter wavelength relates to smaller ones of the blood vessels than a longer wavelength.

5. An apparatus according to claim 3 or 4, wherein the control system (103) is configured to compute a ratio of at least one pair of the coefficients of time corresponding to different wavelengths, each ratio expressing a stiffness mismatch between ones of the blood vessels having different sizes where a shorter wavelength relates to smaller ones of the blood vessels than a longer wavelength.

6. An apparatus according to any one of claims 1-5, wherein the control system (103) is configured to convert the measurement signal to a logarithmic scale, to find from the converted measurement signal a portion whose envelope has linear change with respect to time, and to produce an estimate for a slope (α_{IR}, α_{R}, α_{Y}, α_{G}, α_{B}) of the envelope of the converted measurement signal related to the linear change, the slope being the coefficient of time related to the exponential change.

7. An apparatus according to any one of claims 1-6, wherein the pressure instrument (102) comprises a force generator (104) and a pressing element (105) configured to direct the mechanical pressure to a fingertip or a toe in accordance with a control signal generated by the control system (103).

8. An apparatus according to any one of claims 1-7, wherein the pressure instrument (202) comprises a cuff and a pump system (242) configured to control gas pressure inside the cuff to direct the mechanical pressure to an arm and to change the mechanical pressure when the photoplethysmography sensor (201) emits and receives the electromagnetic radiation to and from the arm, the photoplethysmography sensor being located on an inner surface of the cuff.

9. A method for measuring compliance of blood vessels, the method comprising:
- emitting (301) electromagnetic radiation to the blood vessels,
- receiving (302) a part of the electromagnetic radiation reflected off the blood vessels,
- producing (303) a measurement signal indicative of the received part of the electromagnetic radiation, and
- producing (304) mechanical pressure applied on the blood vessels and changing linearly with respect to time (t) during the emitting the electromagnetic radiation to the blood vessels and the receiving the part of the electromagnetic radiation reflected off the blood vessels,
**characterized in that** the method comprises finding (305), from the measurement signal, a portion whose envelope has exponential change (~e^{αt} or ~e^{-αt}) with respect to time and producing (306) an estimate for a coefficient (α) of time related to the exponential change, the coefficient of time being indicative of the compliance of the blood vessels, the exponential change being exponential increase when the mechanical pressure is linearly decreased with respect to time, and the exponential change being exponential decrease when the mechanical pressure is linearly increased with respect to time.

10. A method according to claim 9, wherein the electromagnetic radiation has wavelengths selected from at least one of the following ranges: from 625 nm to 1000 nm, from 565 nm to 590 nm, from 500 nm to 565 nm, and from 450 nm to 485 nm.

11. A method according to claim 9 or 10, wherein the electromagnetic radiation has different wavelengths and the measurement signal comprises wavelength-specific component signals being indicative of received wavelengths reflected off the blood vessels, and the coefficient of time is produced for each of the wavelength-specific component signals corresponding to the different wavelengths where a shorter one of the wavelengths relates to smaller ones of the blood vessels than a longer one of the wavelengths.

12. A method according to claim 9 or 10, wherein wavelength of the electromagnetic radiation is varied, and the coefficient of time is produced for each value of the wavelength where a shorter wavelength relates to smaller ones of the blood vessels than a longer wavelength.

13. A method according to claim 11 or 12, wherein the method comprises computing a ratio of at least one pair of the coefficients of time corresponding to different wavelengths, each ratio expressing a stiffness mismatch between ones of the blood vessels having different sizes where a shorter wavelength relates to smaller ones of the blood vessels than a longer wavelength.

14. A computer program for measuring compliance of blood vessels, the computer program comprising computer executable instructions for controlling a programmable processing system to:
- control a photoplethysmography sensor to emit electromagnetic radiation to the blood vessels, to receive a part of the electromagnetic radiation reflected off the blood vessels, and to produce a measurement signal indicative of the received part of the electromagnetic radiation,
- control a pressure instrument to produce mechanical pressure applied on the blood vessels and to change the mechanical pressure linearly with respect to time (t) during emission of the electromagnetic radiation to the blood vessels and reception of the part of the electromagnetic radiation reflected off the blood vessels,
**characterized in that** the computer program further comprises computer executable instructions for controlling the programmable processing system to find, from the measurement signal, a portion whose envelope has exponential change (~e^{αt} or ~e^{-αt}) with respect to time and to produce an estimate for a coefficient (α) of time related to the exponential change, the coefficient of time being indicative of the compliance of the blood vessels, the exponential change being exponential increase when the mechanical pressure is linearly decreased with respect to time, and the exponential change being exponential decrease when the mechanical pressure is linearly increased with respect to time.

15. A computer program product comprising a non-transitory computer readable medium encoded with a computer program according to claim 14.

## Patentansprüche

1. Vorrichtung zum Messen der Nachgiebigkeit von Blutgefäßen, wobei die Vorrichtung umfasst:
- einen Photoplethysmographiesensor (101, 201), der konfiguriert ist, um elektromagnetische Strahlung an die Blutgefäße auszusenden, um einen Teil der von den Blutgefäßen reflektierten elektromagnetischen Strahlung zu empfangen und um ein Messsignal (122) als Indikator für den empfangenen Anteil der elektromagnetischen Strahlung zu erzeugen,
- ein Druckinstrument (102, 202), das konfiguriert ist, um einen auf die Blutgefäße ausgeübten mechanischen Druck zu erzeugen, und
- ein Steuerungssystem (103, 203), das konfiguriert ist, um das Druckinstrument zu steuern, um den mechanischen Druck während der Aussendung der elektromagnetischen Strahlung an die Blutgefäße und des Empfangens des von den Blutgefäßen reflektierten Teils der elektromagnetischen Strahlung linear im Verhältnis zur Zeit (t) zu ändern,
**dadurch gekennzeichnet, dass** das Steuerungssystem konfiguriert ist, um einen Abschnitt aus dem Messsignal zu finden, dessen Hüllkurve sich im Verhältnis zur Zeit exponentiell ändert (~e^{αt} oder ~e^{-αt}) und um eine Schätzung für einen Koeffizienten (α) der Zeit zu erzeugen, der mit der exponentiellen Änderung in Zusammenhang steht, wobei der Zeitkoeffizient ein Indikator für die Nachgiebigkeit der Blutgefäße ist, wobei es sich bei der exponentiellen Änderung um eine exponentielle Erhöhung handelt, wenn der mechanische Druck im Verhältnis zur Zeit linear verringert wird, und wobei es sich bei der exponentiellen Änderung um eine exponentielle Verringerung handelt, wenn der mechanische Druck im Verhältnis zur Zeit linear erhöht wird.

2. Vorrichtung nach Anspruch 1, wobei der Photoplethysmographiesensor (101) konfiguriert ist, um die elektromagnetische Strahlung derart an die Blutgefäße auszusenden, dass die elektromagnetische Strahlung Wellenlängen aufweist, die aus mindestens einem der folgenden Bereiche ausgewählt sind: von 625 nm bis 1000 nm, von 565 nm bis 590 nm, von 500 nm bis 565 nm und von 450 nm bis 485 nm.

3. Vorrichtung nach Anspruch 1 oder 2, wobei der Photoplethysmographiesensor (101) konfiguriert ist, um die elektromagnetische Strahlung derart an die Blutgefäße auszusenden, dass die elektromagnetische Strahlung unterschiedliche Wellenlängen aufweist, und um das Messsignal (122) zu erzeugen, das wellenlängenspezifische Komponentensignale (117-121) als Indikatoren für die empfangenen, von den Blutgefäßen reflektierten Wellenlängen umfasst, und wobei das Steuerungssystem konfiguriert ist, um den Zeitkoeffizienten für jedes der wellenlängenspezifischen Komponentensignale zu erzeugen, die den unterschiedlichen Wellenlängen entsprechen, wobei sich eine kürzere der Wellenlängen auf kleinere Blutgefäße bezieht als eine längere der Wellenlängen.

4. Vorrichtung nach Anspruch 1 oder 2, wobei das Steuerungssystem (103) konfiguriert ist, um den Photoplethysmographiesensor (101) zu steuern, um die Wellenlänge der elektromagnetischen Strahlung zu variieren und den Zeitkoeffizienten für jeden Wert der Wellenlänge zu erzeugen, wobei sich eine kürzere Wellenlänge auf kleinere Blutgefäße bezieht als eine längere Wellenlänge.

5. Vorrichtung nach Anspruch 3 oder 4, wobei das Steuerungssystem (103) konfiguriert ist, um ein Verhältnis von mindestens einem Paar von Zeitkoeffizienten entsprechend unterschiedlichen Wellenlängen zu berechnen, wobei jedes Verhältnis einen Steifigkeitssprung zwischen Blutgefäßen unterschiedlicher Größe ausdrückt, wobei sich eine kürzere Wellenlänge auf kleinere Blutgefäße bezieht als eine längere Wellenlänge.

6. Vorrichtung nach einem der Ansprüche 1 bis 5, wobei das Steuerungssystem (103) konfiguriert ist, um das Messsignal in eine logarithmische Skala umzurechnen, aus dem umgerechneten Messsignal einen Abschnitt zu finden, dessen Hüllkurve sich im Verhältnis zur Zeit linear ändert, und einen Schätzwert für eine Steigung (α_{IR}, α_{R}, α_{γ}, α_{G}, α_{B}) der Hüllkurve des umgerechneten Messsignals in Bezug auf die lineare Änderung zu erzeugen, wobei die Steigung der Zeitkoeffizient im Zusammenhang mit der exponentiellen Änderung ist.

7. Vorrichtung nach einem der Ansprüche 1 bis 6, wobei das Druckinstrument (102) einen Kraftgenerator (104) und ein Druckelement (105) umfasst, das konfiguriert ist, um den mechanischen Druck gemäß einem von dem Steuerungssystem (103) erzeugten Steuersignal zu einer Fingerspitze oder einem Zeh zu leiten.

8. Vorrichtung nach einem der Ansprüche 1 bis 7, wobei das Druckinstrument (202) eine Manschette und ein Pumpensystem (242) umfasst, das konfiguriert ist, um den Gasdruck innerhalb der Manschette zu steuern, um den mechanischen Druck zu einem Arm zu leiten und den mechanischen Druck zu ändern, wenn der Photoplethysmographiesensor (201) die elektromagnetische Strahlung zu dem Arm aussendet und von dem Arm empfängt, wobei sich der Photoplethysmographiesensor an einer Innenfläche der Manschette befindet.

9. Verfahren zum Messen der Nachgiebigkeit von Blutgefäßen, wobei das Verfahren umfasst:
- Aussenden (301) elektromagnetischer Strahlung an die Blutgefäße,
- Empfangen (302) eines Teils der von den Blutgefäßen reflektierten elektromagnetischen Strahlung,
- Erzeugen (303) eines Messsignals als Indikator für den empfangenen Anteil der elektromagnetischen Strahlung und
- Erzeugen (304) eines auf die Blutgefäße ausgeübten Drucks und dessen lineare Änderung im Verhältnis zur Zeit (t) während der Aussendung der elektromagnetischen Strahlung an die Blutgefäße und des Empfangens des von den Blutgefäßen reflektierten Teils der elektromagnetischen Strahlung,
**dadurch gekennzeichnet, dass** das Verfahren das Finden (305) eines Abschnitts aus dem Messsignal, dessen Hüllkurve sich im Verhältnis zur Zeit exponentiell ändert (~e^{αt} oder ~e^{-αt}), und das Erzeugen (306) einer Schätzung für einen Koeffizienten (α) der Zeit, der mit der exponentiellen Änderung in Zusammenhang steht, umfasst, wobei der Zeitkoeffizient ein Indikator für die Nachgiebigkeit der Blutgefäße ist, wobei es sich bei der exponentiellen Änderung um eine exponentielle Erhöhung handelt, wenn der mechanische Druck im Verhältnis zur Zeit linear verringert wird, und wobei es sich bei der exponentiellen Änderung um eine exponentielle Verringerung handelt, wenn der mechanische Druck im Verhältnis zur Zeit linear erhöht wird.

10. Verfahren nach Anspruch 9, wobei die elektromagnetische Strahlung Wellenlängen aufweist, die aus mindestens einem der folgenden Bereiche ausgewählt sind: von 625 nm bis 1000 nm, von 565 nm bis 590 nm, von 500 nm bis 565 nm und von 450 nm bis 485 nm.

11. Verfahren nach Anspruch 9 oder 10, wobei die elektromagnetische Strahlung unterschiedliche Wellenlängen aufweist und das Messsignal wellenlängenspezifische Komponentensignale als Indikatoren für die empfangenen, von den Blutgefäßen reflektierten Wellenlängen umfasst, und der Zeitkoeffizient für jedes der wellenlängenspezifischen Komponentensignale erzeugt wird, die den unterschiedlichen Wellenlängen entsprechen, wobei sich eine kürzere der Wellenlängen auf kleinere Blutgefäße bezieht als eine längere der Wellenlängen.

12. Verfahren nach Anspruch 9 oder 10, wobei die Wellenlänge der elektromagnetischen Strahlung variiert wird und der Zeitkoeffizient für jeden Wert der Wellenlänge erzeugt wird, wobei sich eine kürzere Wellenlänge auf kleinere Blutgefäße bezieht als eine längere Wellenlänge.

13. Verfahren nach Anspruch 11 oder 12, wobei das Verfahren das Berechnen eines Verhältnisses von mindestens einem Paar von Zeitkoeffizienten entsprechend unterschiedlichen Wellenlängen umfasst, wobei jedes Verhältnis einen Steifigkeitssprung zwischen Blutgefäßen unterschiedlicher Größe ausdrückt, wobei sich eine kürzere Wellenlänge auf kleinere Blutgefäße bezieht als eine längere Wellenlänge.

14. Computerprogramm zum Messen der Nachgiebigkeit von Blutgefäßen, wobei das Computerprogramm computerausführbare Anweisungen zum Steuern eines programmierbaren Verarbeitungssystems umfasst, um:
- einen Photoplethysmographiesensor zu steuern, um elektromagnetische Strahlung an die Blutgefäße auszusenden, um einen Teil der von den Blutgefäßen reflektierten elektromagnetischen Strahlung zu empfangen und um ein Messsignal als Indikator für den empfangenen Anteil der elektromagnetischen Strahlung zu erzeugen,
- ein Druckinstrument zu steuern, um einen auf die Blutgefäße ausgeübten mechanischen Druck zu erzeugen und den mechanischen Druck während der Aussendung der elektromagnetischen Strahlung an die Blutgefäße und des Empfangens des von den Blutgefäßen reflektierten Teils der elektromagnetischen Strahlung linear im Verhältnis zur Zeit (t) zu ändern,
**dadurch gekennzeichnet, dass** das Computerprogramm ferner computerausführbare Anweisungen zum Steuern des programmierbaren Verarbeitungssystems umfasst, um einen Abschnitt aus dem Messsignal zu finden, dessen Hüllkurve sich im Verhältnis zur Zeit exponentiell ändert (~e^{αt} oder ~e^{-αt}), und um eine Schätzung für einen Koeffizienten (α) der Zeit zu erzeugen, der mit der exponentiellen Änderung in Zusammenhang steht, wobei der Zeitkoeffizient ein Indikator für die Nachgiebigkeit der Blutgefäße ist, wobei es sich bei der exponentiellen Änderung um eine exponentielle Erhöhung handelt, wenn der mechanische Druck im Verhältnis zur Zeit linear verringert wird, und wobei es sich bei der exponentiellen Änderung um eine exponentielle Verringerung handelt, wenn der mechanische Druck im Verhältnis zur Zeit linear erhöht wird.

15. Computerprogrammprodukt umfassend ein nichtflüchtiges computerlesbares Medium, das mit einem Computerprogramm gemäß Anspruch 14 codiert ist.

## Revendications

1. Appareil pour mesurer l'élasticité de vaisseaux sanguins, l'appareil comprenant :
- un capteur de photopléthysmographie (101, 201) configuré pour émettre un rayonnement électromagnétique vers les vaisseaux sanguins, pour recevoir une partie du rayonnement électromagnétique réfléchi par les vaisseaux sanguins, et pour produire un signal de mesure (122) indiquant la partie reçue du rayonnement électromagnétique,
- un instrument de pression (102, 202) configuré pour produire une pression mécanique appliquée sur les vaisseaux sanguins, et
- un système de commande (103, 203) configuré pour commander l'instrument de pression à changer la pression mécanique de manière linéaire par rapport au temps (t) pendant l'émission du rayonnement électromagnétique vers les vaisseaux sanguins et la réception de la partie du rayonnement électromagnétique réfléchi par les vaisseaux sanguins,
**caractérisé en ce que** le système de commande est configuré pour trouver, à partir du signal de mesure, une partie dont l'enveloppe présente un changement exponentiel (~e^{αt} ou ~e^{αt}) par rapport au temps et pour produire une estimation d'un coefficient (α) de temps lié au changement exponentiel, le coefficient de temps indiquant l'élasticité des vaisseaux sanguins, le changement exponentiel étant une augmentation exponentielle lorsque la pression mécanique est baissée de manière linéaire par rapport au temps, et le changement exponentiel étant une baisse exponentielle lorsque la pression mécanique est augmentée de manière linéaire par rapport au temps.

2. Appareil selon la revendication 1, dans lequel le capteur de photopléthysmographie (101) est configuré pour émettre, vers les vaisseaux sanguins, le rayonnement électromagnétique de façon à ce que le rayonnement électromagnétique présente des longueurs d'onde sélectionnées à partir d'au moins une des plages suivantes : de 625 nm à 1000 nm, de 565 nm à 590 nm, de 500 nm à 565 nm et de 450 nm à 485 nm.

3. Appareil selon la revendication 1 ou 2, dans lequel le capteur de photopléthysmographie (101) est configuré pour émettre, vers les vaisseaux sanguins, le rayonnement électromagnétique de façon à ce que le rayonnement électromagnétique présente des longueurs d'onde différentes et pour produire le signal de mesure (122) pour comprendre des signaux de composante spécifique à la longueur d'onde (117-121) indiquant des longueurs d'onde reçues réfléchies par les vaisseaux sanguins, et le système de commande est configuré pour produire le coefficient de temps pour chacun des signaux de composante spécifique à la longueur d'onde correspondant aux différentes longueurs d'onde où une plus courte des longueurs d'onde est liée à des plus petits des vaisseaux sanguins qu'une plus longue des longueurs d'onde.

4. Appareil selon la revendication 1 ou 2, dans lequel le système de commande (103) est configuré pour commander le capteur de photopléthysmographie (101) à varier la longueur d'onde du rayonnement électromagnétique et pour produire le coefficient de temps pour chaque valeur de la longueur d'onde où une longueur d'onde plus courte est liée à des plus petits des vaisseaux sanguins qu'une longueur d'onde plus grande.

5. Appareil selon la revendication 3 ou 4, dans lequel le système de commande (103) est configuré pour calculer un rapport d'au moins une paire des coefficients de temps correspondant à différentes longueurs d'onde, chaque rapport exprimant un décalage de raideur entre des vaisseaux sanguins ayant différentes tailles où une longueur d'onde plus courte est liée à des plus petits des vaisseaux sanguins qu'une longueur d'onde plus longue.

6. Appareil selon l'une quelconque de revendications 1-5, dans lequel le système de commande (103) est configuré pour convertir le signal de mesure à une échelle logarithmique, pour trouver à partir du signal de mesure converti une partie dont l'enveloppe présente un changement linéaire par rapport au temps, et pour produire une estimation d'une pente (α_{IR}, α_{R}, α_{γ}, α_{G}, α_{B}) de l'enveloppe du signal de mesure converti lié au changement linéaire, la pente étant le coefficient de temps lié au changement exponentiel.

7. Appareil selon l'une quelconque de revendications 1-6, dans lequel l'instrument de pression (102) comprend un générateur de force (104) et un élément de pressage (105) configurés pour diriger la pression mécanique sur un bout de doigt ou un orteil en fonction d'un signal de commande généré par le système de commande (103).

8. Appareil selon l'une quelconque de revendications 1-7, dans lequel l'instrument de pression (202) comprend une manchette et un système de pompe (242) configuré pour commander de la pression de gaz à l'intérieur de la manchette pour diriger la pression mécanique sur un bras et pour changer la pression mécanique lorsque le capteur de photopléthysmographie (201) émet et reçoit le rayonnement électromagnétique vers et à partir du bras, le capteur de photopléthysmographie étant situé sur une surface intérieure de la manchette.

9. Procédé pour mesurer l'élasticité de vaisseaux sanguins, le procédé comprenant :
- d'émettre (301) un rayonnement électromagnétique vers les vaisseaux sanguins,
- de recevoir (302) une partie du rayonnement électromagnétique réfléchi par les vaisseaux sanguins,
- de produire (303) un signal de mesure indiquant la partie reçue du rayonnement électromagnétique, et
- de produire (304) de la pression mécanique appliquée sur les vaisseaux sanguins et de changer de manière linéaire par rapport au temps (t) pendant l'émission le rayonnement électromagnétique vers les vaisseaux sanguins et durant la réception la partie du rayonnement électromagnétique réfléchie par les vaisseaux sanguins,
**caractérisé en ce que** le procédé comprend de trouver (305), à partir du signal de mesure, une partie dont l'enveloppe présente un changement exponentiel (~e^{αt} ou ~e^{αt}) par rapport au temps et de produire (306) une estimation d'un coefficient (α) de temps lié au changement exponentiel, le coefficient de temps indiquant l'élasticité des vaisseaux sanguins, le changement exponentiel étant une augmentation exponentielle lorsque la pression mécanique est baissée de manière linéaire par rapport au temps, et le changement exponentiel étant une baisse exponentielle lorsque la pression mécanique est augmentée de manière linéaire par rapport au temps.

10. Procédé selon la revendication 9, dans lequel le rayonnement électromagnétique présente des longueurs d'onde sélectionnées à partir d'au moins une des plages suivantes : de 625 nm à 1000 nm, de 565 nm à 590 nm, de 500 nm à 565 nm et de 450 nm à 485 nm.

11. Procédé selon la revendication 9 ou 10, dans lequel le rayonnement électromagnétique présente des longueurs d'onde différentes et le signal de mesure comprend des signaux de composante spécifique à la longueur d'onde indiquant des longueurs d'onde reçues réfléchies par les vaisseaux sanguins, et le coefficient de temps est produit pour chacun des signaux de composante spécifique à la longueur d'onde correspondant aux différentes longueurs d'onde où une plus courte des longueurs d'onde est liée à des plus petits des vaisseaux sanguins qu'une plus longue des longueurs d'onde.

12. Procédé selon la revendication 9 ou 10, dans lequel la longueur d'onde du rayonnement électromagnétique varie, et le coefficient de temps est produit pour chaque valeur de la longueur d'onde où une longueur d'onde plus courte est liée à des plus petits des vaisseaux sanguins qu'une longueur d'onde plus longue.

13. Procédé selon la revendication 11 ou 12, dans lequel le procédé comprend de calculer un rapport d'au moins une paire des coefficients de temps correspondant à différentes longueurs d'onde, chaque rapport exprimant un décalage de raideur entre des vaisseaux sanguins ayant différentes tailles où une longueur d'onde plus courte est liée à des plus petits des vaisseaux sanguins qu'une longueur d'onde plus longue.

14. Programme informatique pour mesurer l'élasticité de vaisseaux sanguins, le programme informatique comprenant des instructions pouvant être exécutées sur ordinateur pour commander un système de traitement programmable à :
- commander un capteur de photopléthysmographie à émettre un rayonnement électromagnétique vers les vaisseaux sanguins, pour recevoir une partie du rayonnement électromagnétique réfléchi par les vaisseaux sanguins, et pour produire un signal de mesure indiquant la partie reçue du rayonnement électromagnétique,
- commander un instrument de pression à produire une pression mécanique appliquée sur les vaisseaux sanguins, et à changer la pression mécanique de manière linéaire par rapport au temps (t) pendant l'émission du rayonnement électromagnétique vers les vaisseaux sanguins et la réception de la partie du rayonnement électromagnétique réfléchi par les vaisseaux sanguins,
**caractérisé en ce que** le programme informatique comprend en outre des instructions pouvant être exécutées sur un ordinateur pour commander le système de traitement programmable à trouver, à partir du signal de mesure, une partie dont l'enveloppe présente un changement exponentiel (~e^{αt} ou ~e^{αt}) par rapport au temps et pour produire une estimation d'un coefficient (α) de temps lié au changement exponentiel, le coefficient de temps indiquant l'élasticité des vaisseaux sanguins, le changement exponentiel étant une augmentation exponentielle lorsque la pression mécanique est baissée de manière linéaire par rapport au temps, et le changement exponentiel étant une baisse exponentielle lorsque la pression mécanique est augmentée de manière linéaire par rapport au temps.

15. Produit de programme informatique comprenant un support lisible sur ordinateur non-transitoire encodé avec un programme informatique selon la revendication 14.
